(19) Europäisches Patentamt / European Patent Office / Office européen des brevets

(11) **EP 2 469 440 A2**

(12) **EUROPEAN PATENT APPLICATION**

(43) Date of publication:
    **27.06.2012 Bulletin 2012/26**

(51) Int Cl.:
    **G06F 19/24** (2011.01)      *C12Q 1/68* (2006.01)

(21) Application number: **11193726.4**

(22) Date of filing: **16.06.2009**

(84) Designated Contracting States:
    **AT BE BG CH CY CZ DE DK EE ES FI FR GB GR HR HU IE IS IT LI LT LU LV MC MK MT NL NO PL PT RO SE SI SK TR**

(30) Priority: **16.06.2008 EP 08010919**

(62) Document number(s) of the earlier application(s) in accordance with Art. 76 EPC:
    **09779774.0 / 2 304 630**

(27) Previously filed application:
    **16.06.2009 EP 09779774**

(71) Applicant: **Sividon Diagnostics GmbH**
    **50829 Köln (DE)**

(72) Inventors:
    • **Gehrmann, Mathias**
      **51375 Leverkusen (DE)**

    • **Stropp, Udo**
      **42781 Haan (DE)**
    • **Weber, Karsten**
      **51379 Leverkusen (DE)**
    • **von Törne, Christian**
      **42659 Solingen (DE)**
    • **Schmidt, Marcus**
      **55252 Mainz-Kastel (DE)**

(74) Representative: **von Kreisler Selting Werner**
    **Deichmannhaus am Dom**
    **Bahnhofsvorplatz 1**
    **50667 Köln (DE)**

Remarks:
    This application was filed on 15-12-2011 as a divisional application to the application mentioned under INID code 62.

(54) **Molecular markers for cancer prognosis**

(57)    The present invention relates to methods for prediction of an outcome of neoplastic disease or cancer. More specifically, the present invention relates to a method for the prediction of breast cancer by determining in a biological sample from said patient an expression level of a plurality of genes selected from the group consisting of ACTG1, CA12, CALM2, CCND1, CHPT1, CLEC2B, CTSB, CXCL13, DCN, DHRS2, EIF4B, ERBB2, ESR1, FBXO28, GABRP, GAPDH, H2AFZ, IGFBP3, IGHG1, IGKC, KCTD3, KIAA0101, KRT17, MLPH, MMP1, NAT1, NEK2, NR2F2, OAZ1, PCNA, PDLIM5, PGR, PPIA, PRC1, RACGAP1, RPL37A, SOX4, TOP2A, UBE2C and VEGF.

EP 2 469 440 A2

**Description**

The present invention relates to methods for prediction of an outcome of neoplastic disease or cancer. More specifically, the present invention relates to a method for the prediction of breast cancer.

Cancer is a genetically and clinically complex disease with multiple parameters determining outcome and suitable therapy of disease. It is common practice to classify patients into different stages, grades, classes of disease status and the like and to use such classification to predict disease outcome and for choice of therapy options. It is for example desirable to be able to predict a risk of recurrence of disease, risk of metastasis and the like.

Breast Cancer (BRC) is the leading cause of death in women between ages of 35 - 55. Worldwide, there are over 3 million women living with breast cancer. OECD (Organization for Economic Cooperation & Development) estimates on a worldwide basis 500,000 new cases of breast cancer are diagnosed each year. One out of ten women will face the diagnosis breast cancer at some point during her lifetime.

Breast cancer is the abnormal growth of cells that line the breast tissue ducts and lobules and is classified by whether the cancer started in the ducts or the lobules and whether the cells have invaded (grown or spread) through the duct or lobule, and by the way the cells look under the microscope (tissue histology). It is not unusual for a single breast tumor to have a mixture of invasive and in situ cancer. According to today's therapy guidelines and current medical practice, the selection of a specific therapeutic intervention is mainly based on histology, grading, staging and hormonal status of the patient. Many aspects of a patient's specific type of tumor are currently not assessed - preventing true patient-tailored treatment. Another dilemma of today's breast cancer therapeutic regimens is the practice of significant over-treatment of patients; it is well known from past clinical trials that 70% of breast cancer patients with early stage disease do not need any treatment beyond surgery. While about 90% of all early stage cancer patients receive chemotherapy exposing them to significant treatment side effects, approximately 30% of patients with early stage breast cancer relapse. As such, there is a significant medical need to develop diagnostic assays that identify low risk patients for directed therapy. For patients with medium or high risk assessment, there is a need to pinpoint therapeutic regimens tailored to the specific cancer to assure optimal success.

Breast Cancer metastasis and disease-free survival prediction or the prediction of overall survival is a challenge for all pathologists and treating oncologists. A test that can predict such features has a high medical and diagnostical need. We describe here a set of genes that can predict with the help of an algorithm the prognosis of a breast cancer patient of getting a metastasis within 5 or 10 years after surgical removal of the tumor (high risk) or getting no metastasis (low risk or good prognosis). Surprisingly we found that the endpoint "death after recurrence" can also be perfectly predicted with the here described algorithms. Other endpoints can be predicted as well, like overall and disease-free survival, or death of disease and others.

This disclosure focuses on a breast cancer prognosis test as a comprehensive predictive breast cancer marker panel for lymph node-negative breast cancer patients. About 80% of all breast cancers diagnosed in the US and Europe are node-negative. The prognostic test will stratify diagnosed lymph node-negative breast cancer patients into low, (medium) or high risk groups according to a continuous score that will be generated by the algorithms. One or two cutpoints will classify the patients according to their risk (low, (medium) or high). The stratification will provide the treating oncologist with the likelihood that the tested patient will suffer from cancer recurrence in the absence of therapy. The oncologist can utilize the results of this test to make decisions on therapeutic regimens. Although the test is useful for reducing overtreatment according to current therapy guidelines the test can be used to find optimal therapies especially but not exclusively for patients with medium or high risk.

The metastatic potential of primary tumors is the chief prognostic determinant of malignant disease. Therefore, predicting the risk of a patient developing metastasis is an important factor in predicting the outcome of disease and choosing an appropriate treatment.

As an example, breast cancer is the leading cause of death in women between the ages of 35-55. Worldwide, there are over 3 million women living with breast cancer. OECD (Organization for Economic Cooperation & Development) estimates on a worldwide basis 500,000 new cases of breast cancer are diagnosed each year. One out of ten women will face the diagnosis breast cancer at some point during her lifetime. Breast cancer is the abnormal growth of cells that line the breast tissue ducts and lobules and is classified by whether the cancer started in the ducts or the lobules and whether the cells have invaded (grown or spread) through the duct or lobule, and by the way the cells appear under the microscope (tissue histology). It is not unusual for a single breast tumor to have a mixture of invasive and in situ cancer. According to today's therapy guidelines and current medical practice, the selection of a specific therapeutic intervention is mainly based on histology, grading, staging and hormonal status of the patient. Many aspects of a patient's specific type of tumor are currently not assessed - preventing true patient-tailored treatment. Another dilemma of today's breast cancer therapeutic regimens is the practice of significant over-treatment of patients; it is well known from past clinical trials that 70% of breast cancer patients with early stage disease do not need any treatment beyond surgery. While about 90% of all early stage cancer patients receive chemotherapy exposing them to significant treatment side effects, approximately 30% of patients with early stage breast cancer relapse. These types of problems are common to other forms of cancer as well. As such, there is a significant medical need to develop diagnostic assays that identify low risk patients for directed therapy. For patients with medium or high risk assessment, there is a need to pinpoint therapeutic regimens tailored to the specific cancer to assure optimal success. Breast Cancer metastasis and disease-free survival prediction is a challenge for all pathologists and treating oncologists. A test that can predict such features has a high medical and diagnostic need.

About 80% of all breast cancers diagnosed in the US and Europe are node-negative. What is needed are diagnostic tests and methods which can assess certain disease-related risks, e.g. risk of development of metastasis.

Technologies such as quantitative PCR, microarray analysis, and others allow the analysis of genome-wide expression patterns which provide new insight into gene regulation and are also a useful diagnostic tool because they allow the analysis of pathologic conditions at the level of gene expression. Quantitative reverse transcriptase PCR is currently the accepted standard for quantifying gene expression. It has the advantage of being a very sensitive method allowing the detection of even minute amounts of mRNA. Microarray analysis is fast becoming a new standard for quantifying gene expression.

Curing breast cancer patients is still a challenge for the treating oncologist as the diagnosis relies in most cases on clinical data such as etiopathological and pathological data like age, menopausal status, hormonal status, grading, and general constitution of the patient, and some molecular markers like Her2/neu, p53, and some others. Unfortunately, until recently, there was no test in the market for prognosis or therapy prediction that comes up with a more elaborated recommendation for the treating oncologist whether and how to treat patients. Two assay systems are currently available for prognosis, Genomic Health's OncotypeDX and Agendia's Mammaprint assay. In 2007, the company Agendia got FDA approval for their Mammaprint microarray assay that can predict with the help of 70 informative genes and a bundle of housekeeping genes the prognosis of breast cancer patients from fresh tissue (Glas A.M. et al., Converting a breast cancer microarray signature into a high-throughput diagnostic test, BMC Genomics. 2006 Oct 30; 7:278). The Genomic Health assay works with formalin-fixed and paraffin-embedded tumor tissue samples and uses 21 genes for the prognosis, presented as a risk score (Esteva FT et al. Prognostic role of a multigene reverse transcriptase-PCR assay in patients with node-negative breast cancer not receiving adjuvant systemic therapy. Clin Cancer Res 2005; 11: 3315-3319).

Both these assays use a high number of different markers to arrive at a result and require a high number of internal controls to ensure accurate results. What is needed is a simple and robust assay for prediction and/or prognosis of cancer.

Objective of the invention

It is an objective of the invention to provide a method for the prediction and/or prognosis of cancer relying on a limited number of markers.

It is a further objective of the invention to provide a kit for performing the method of the invention.

Definitions

The term "neoplastic disease", "neoplastic region", or "neoplastic tissue" refers to a tumorous tissue including carcinoma (e.g. carcinoma in situ, invasive carcinoma, metastasis carcinoma) and pre-malignant conditions, neomorphic changes independent of their histological origin, cancer, or cancerous disease.

The term "cancer" is not limited to any stage, grade, histomorphological feature, aggressivity, or malignancy of an affected tissue or cell aggregation. In particular, solid tumors, malignant lymphoma and all other types of cancerous tissue, malignancy and transformations associated therewith, lung cancer, ovarian cancer, cervix cancer, stomach cancer, pancreas cancer, prostate cancer, head and neck cancer, renal cell cancer, colon cancer or breast cancer are included. The terms "neoplastic lesion" or "neoplastic disease" or "neoplasm" or "cancer" are not limited to any tissue or cell type. They also include primary, secondary, or metastatic lesions of cancer patients, and also shall comprise lymph nodes affected by cancer cells or minimal residual disease cells either locally deposited or freely floating throughout the patient's body.

The term "predicting an outcome" of a disease, as used herein, is meant to include both a prediction of an outcome of a patient undergoing a given therapy and a prognosis of a patient who is not treated. The term "predicting an outcome" may, in particular, relate to the risk of a patient developing metastasis, local recurrence or death.

The term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assessment of the malignancy of a tumor, or to the expected survival rate (DFS, disease free survival) of a patient, if the tumor remains untreated.

A "discriminant function" is a function of a set of variables used to classify an object or event. A discriminant function thus allows classification of a patient, sample or event into a category or a plurality of categories according to data or parameters available from said patient, sample or event. Such classification is a standard instrument of statistical analysis well known to the skilled person. E.g. a patient may be classified as "high risk" or "low risk", "high probability of metastasis" or "low probability of metastasis", "in need of treatment" or "not in need of treatment" according to data obtained from said patient, sample or event. Classification is not limited to "high vs. low", but may be performed into a plurality categories, grading or the like. Classification shall also be understood in a wider sense as a discriminating score, where e.g. a higher score represents a higher likelihood of distant metastasis, e.g. the (overall) risk of a distant metastasis. Examples for discriminant functions which allow a classification include, but are not limited to functions defined by support vector machines (SVM), k-nearest neighbors (kNN), (naive) Bayes models, linear regression models, or piecewise defined functions such as, for example, in subgroup discovery, in decision trees, in logical analysis of data (LAD) and the like. In a wider sense, continuous score values of mathematical methods or algorithms, such as correlation coefficients, projections, support vector machine scores, other similarity-based methods, combinations of these and the like are examples for illustrative purpose.

An "outcome" within the meaning of the present invention is a defined condition attained in the course of the disease. This disease outcome may e.g. be a clinical condition such as "recurrence of disease", "development of metastasis", "development of nodal metastasis", development of distant metastasis", "survival", "death", "tumor remission rate", a disease stage or grade or the like.

A "risk" is understood to be a probability of a subject or a patient to develop or arrive at a certain disease outcome. The term "risk" in the context of the present invention is not meant to carry any positive or negative connotation with regard to a patient's wellbeing but merely refers to a probability or likelihood of an occurrence or development of a given condition.

The term "clinical data" relates to the entirety of available data and information concerning the health status of a patient including, but not limited to, age, sex, weight, menopausal/hormonal status, etiopathology data, anamnesis data, data obtained by in vitro diagnostic methods such as blood or urine tests, data obtained by imaging methods, such as x-ray, computed tomography, MRI, PET, spect, ultrasound, electrophysiological data, genetic analysis, gene expression analysis, biopsy evaluation, intraoperative findings.

The term "etiopathology" relates to the course of a disease, that is its duration, its clinical symptoms, signs and parameters, and its outcome.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

In the context of the present invention a "biological sample" is a sample which is derived from or has been in contact with a biological organism. Examples for biological samples are: cells, tissue, body fluids, lavage fluid, smear samples, biopsy specimens, blood, urine, saliva, sputum, plasma, serum, cell culture supernatant, and others.

A "biological molecule" within the meaning of the present invention is a molecule generated or produced by a biological organism or indirectly derived from a molecule generated by a biological organism, including, but not limited to, nucleic acids, protein, polypeptide, peptide, DNA, mRNA, cDNA, and so on.

A "probe" is a molecule or substance capable of specifically binding or interacting with a specific biological molecule. The term "primer", "primer pair" or "probe", shall have ordinary meaning of these terms which is known to the person skilled in the art of molecular biology. In a preferred embodiment of the invention "primer", "primer pair" and "probes" refer to oligonucleotide or polynucleotide molecules with a sequence identical to, complementary too, homologues of, or homologous to regions of the target molecule or target sequence which is to be detected or quantified, such that the primer, primer pair or probe can specifically bind to the target molecule, e.g. target nucleic acid, RNA, DNA, cDNA, gene, transcript, peptide, polypeptide, or protein to be detected or quantified. As understood herein, a primer may in itself function as a probe. A "probe" as understood herein may also comprise e.g. a combination of primer pair and internal labeled probe, as is common in many commercially available qPCR methods.

A "gene" is a set of segments of nucleic acid that contains the information necessary to produce a functional RNA product. A "gene product" is a biological molecule produced through transcription or expression of a gene, e.g. an mRNA or the translated protein.

An "mRNA" is the transcribed product of a gene and shall have the ordinary meaning understood by a person skilled in the art. A "molecule derived from an mRNA" is a molecule which is chemically or enzymatically obtained from an mRNA template, such as cDNA.

The term "specifically binding" within the context of the present invention means a specific interaction between a probe and a biological molecule leading to a binding complex of probe and biological molecule, such as DNA-DNA binding, RNA-DNA binding, RNA-RNA binding, DNA-protein binding, protein-protein binding, RNA-protein binding, antibody*-antigen binding, and so on.

The term "expression level" refers to a determined level of gene expression. This may be a determined level of gene expression compared to a reference gene (e.g. a housekeeping gene) or to a computed average expression value (e.g. in DNA chip analysis) or to another informative gene without the use of a reference sample. The expression level of a gene may be measured directly, e.g. by obtaining a signal wherein the signal strength is correlated to the amount of mRNA transcripts of that gene or it may be obtained indirectly at a protein level, e.g. by immunohistochemistry, CISH, ELISA or RIA methods. The expression level may also be obtained by way of a competitive reaction to a reference sample.

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

The term "complementary" or "sufficiently complementary" means a degree of complementarity which is - under given assay conditions - sufficient to allow the formation of a binding complex of a primer or probe to a target molecule. Assay conditions which have an influence of binding of probe to target include temperature, solution conditions, such as composition, pH, ion concentrations, etc. as is known to the skilled person.

The term "hybridization-based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are used in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. Hybridization is dependent on target and probe (e.g. length of matching sequence, GC content) and hybridization conditions (temperature, solvent, pH, ion concentrations, presence of denaturing agents, etc.). A "hybridizing counterpart" of a nucleic acid is understood to mean a probe or capture sequence which under given assay conditions hybridizes to said nucleic acid and forms a binding complex with said nucleic acid. Normal conditions refers to temperature and solvent conditions and are understood to mean conditions under which a probe can hybridize to allelic variants of a nucleic acid but does not unspecifically bind to unrelated genes. These conditions are known to the skilled person and are e.g. described in "Molecular Cloning. A laboratory manual", Cold Spring Harbour Laboratory Press, 2. Aufl., 1989. Normal conditions would be e.g. hybridization at 6 x Sodium Chloride/sodium citrate buffer (SSC) at about 45°C, followed by washing or rinsing with 2 x SSC at about 50°C, or e.g. conditions used in standard PCR protocols, such as annealing temperature of 40 to 60°C in standard PCR reaction mix or buffer.

The term "array" refers to an arrangement of addressable locations on a device, e.g. a chip device. The number of locations can range from several to at least hundreds or thousands. Each location represents an independent reaction site. Arrays include, but are not limited to nucleic acid arrays, protein arrays and antibody-arrays. A "nucleic acid array" refers to an array containing nucleic acid probes, such as oligonucleotides, polynucleotides or larger portions of genes. The nucleic acid on the array is preferably single stranded. A "microarray" refers to a biochip or biological chip, i.e. an array of regions having a density of discrete regions with immobilized probes of at least about $100/cm^2$.

A "PCR-based method" refers to methods comprising a polymerase chain reaction PCR. This is a method of exponentially amplifying nucleic acids, e.g. DNA or RNA by enzymatic replication in vitro using one, two or more primers. For RNA amplification, a reverse transcription may be used as a first step. PCR-based methods comprise kinetic or quantitative PCR (qPCR) which is particularly suited for the analysis of expression levels,).

The term "determining a protein level" refers to any method suitable for quantifying the amount, amount relative to a standard or concentration of a given protein in a sample. Commonly used methods to determine the amount of a given protein are e.g. immunohistochemistry, CISH, ELISA or RIA methods. etc.

The term "reacting" a probe with a biological molecule to form a binding complex herein means bringing probe and biologically molecule into contact, for example, in liquid solution, for a time period and under conditions sufficient to form a binding complex.

The term "label" within the context of the present invention refers to any means which can yield or generate or lead to a detectable signal when a probe specifically binds a biological molecule to form a binding complex. This can be a label in the traditional sense, such as enzymatic label, fluorophore, chromophore, dye, radioactive label, luminescent label, gold label, and others. In a more general sense the term "label" herein is meant to encompass any means capable of detecting a binding complex and yielding a detectable signal, which can be detected, e.g. by sensors with optical detection, electrical detection, chemical detection, gravimetric detection (i.e. detecting a change in mass), and others. Further examples for labels specifically include labels commonly used in qPCR methods, such as the commonly used dyes FAM, VIC, TET, HEX, JOE, Texas Red, Yakima Yellow, quenchers like TAMRA, minor groove binder, dark quencher, and others, or probe indirect staining of PCR products by for example SYBR Green. Readout can be performed on hybridization platforms, like Affymetrix, Agilent, Illumina, Planar Wave Guides, Luminex, microarray devices with optical, magnetic, electrochemical, gravimetric detection systems, and others. A label can be directly attached to a probe or indirectly bound to a probe, e.g. by secondary antibody, by biotinstreptavidin interaction or the like.

The term "combined detectable signal" within the meaning of the present invention means a signal, which results, when at least two different biological molecules form a binding complex with their respective probes and one common label yields a detectable signal for either binding event.

A "decision tree" is a is a decision support tool that uses a graph or model of decisions and their possible consequences, including chance event outcomes, resource costs, and utility. A decision tree is used to identify the strategy most likely to reach a goal. Another use of trees is as a descriptive means for calculating conditional probabilities.

In data mining and machine learning, a decision tree is a predictive model; that is, a mapping from observations about an item to conclusions about its target value. More descriptive names for such tree models are classification tree (discrete outcome) or regression tree (continuous outcome). In these tree structures, leaves represent classifications (e.g. "high risk" / "low risk", "suitable for treatment A" / "not suitable for treatment A" and the like), while branches represent conjunctions of features (e.g. features such as "Gene X is strongly expressed compared to a control" vs., "Gene X is weakly expressed compared to a control") that lead to those classifications.

A "fuzzy" decision tree does not rely on yes/no decisions, but rather on numerical values (corresponding e.g. to gene expression values of predictive genes), which then correspond to the likelihood of a certain outcome.

A "motive" is a group of biologically related genes. This biological relation may e.g. be functional (e.g. genes related to the same purpose, such as proliferation, immune response, cell motility, cell death, etc.), the biological relation may also e.g. be a co-regulation of gene expression (e.g. genes regulated by the same or similar transcription factors, promoters or other regulative elements).

Summary of the invention

The invention relates to a method for predicting an outcome of breast cancer in a patient, said method comprising:

(a) determining in a biological sample from said patient an expression level of combination of at least 9 genes said combination comprising CHPT1, CXCL13, ESR1, IGKC, MLPH, MMP1, PGR, RACGAP1, and TOP2A;
(b) based on the expression level of the plurality of genes determined in step (a) determining a risk score for each gene; and
(c) mathematically combining said risk scores to yield a combined score, wherein said combined score is indicative of a prognosis of said patient.

In an embodiment of the invention said combination of at least 9 genes consists of the genes CHPT1, CXCL13, ESR1, IGKC, MLPH, MMP1, PGR, RACGAP1, and TOP2A

In a more general sense, the invention also comprises the methods as defined in the following numbered paragraphs:

1. Method for predicting an outcome of cancer in a patient suffering from or suspected of suffering from neoplastic disease, said method comprising:

(a) determining in a biological sample from said patient an expression level of a plurality of genes selected from the group consisting of ACTG1, CA12, CALM2, CCND1, CHPT1, CLEC2B, CTSB, CXCL13, DCN, DHRS2, EIF4B, ERBB2, ESR1, FBXO28, GABRP, GAPDH, H2AFZ, IGFBP3, IGHG1, IGKC, KCTD3, KIAA0101, KRT17, MLPH, MMP1, NAT1, NEK2, NR2F2, OAZ1, PCNA, PDLIM5, PGR, PPIA, PRC1, RACGAP1, RPL37A, SOX4, TOP2A, UBE2C and VEGF;
(b) based on the expression level of the plurality of genes determined in step (a) determining a risk score for each gene; and
(c) mathematically combining said risk scores to yield a combined score, wherein said combined score is indicative of a prognosis of said patient.

The mathematical combination comprises the use of a discriminant function, e.g. an algorithm, to determine the combined score. Such algorithms may comprise the use of averages, weighted averages, sums, differences, products and/or linear and nonlinear functions to arrive at the combined score. In particular the algorithm may comprise one of the algorithms P1c, P2e, P2e_c, P2e_Mz10, P7a, P7b, P7c, P2e_Mz10_b, and P2e_lin, described below.

The inventors have surprisingly found that using the combination of markers described herein, in particular the combination of CHPT1, CXCL13, ESR1, IGKC, MLPH, MMP1, PGR, RACGAP1, and TOP2A and also using these algorithms give more reliable, statistically significant predictive results while using a smaller number of genes than used in currently available tests as described above. The authors have found a unique combination of genes using genes indicative of hormone receptor status, immune response status, proliferative staus of the tumor, and MMP1, which is indicative of invasive properties of the tumor. These combinations, as referenced in table 2, are particularly well suited for predicting an outcome in cancer patients, especially in breast cancer patients.

2. Method of numbered paragraph 1, wherein one, two or more thresholds are determined for said combined score and discriminated into high and low risk, high, intermediate and low risk, or more risk groups by applying the threshold on the combined score.

3. Method of numbered paragraph 1 or 2, wherein all expression levels in said group of expression levels are determined.

4. Method of any one of the preceding numbered paragraphs wherein said prognosis is the determination of the risk of recurrence of cancer in said patient within 5 to 10 years or the risk of developing distant metastasis within 5 to 10 years, or the prediction of death after recurrence within 5 to 10 years after surgical removal of the tumor.

5. Method of any one of the preceding numbered paragraphs, wherein said prognosis is a classification of said patient into one of three distinct classes, said classes corresponding to a "high risk " class, an "intermediate risk " class and a "low risk" class.

6. Method of any one of the preceding numbered paragraphs, wherein said cancer is breast cancer.

7. Method of any one of the preceding numbered paragraphs, wherein said determination of expression levels is in a formalin-fixed paraffin embedded sample or in a fresh-frozen sample.

8. Method of any one of the preceding numbered paragraphs, comprising the additional steps of:

(d) classifying said sample into one of at least two clinical categories according to clinical data obtained from said patient and/or from said sample, wherein each category is assigned to at least one of said genes of step (a); and
(e) determining for each clinical category a risk score; wherein said combined score is obtained by mathematically combining said risk scores of each patient.

9. Method of numbered paragraph 8, wherein said clinical data comprises at least one gene expression level.

10. Method of numbered paragraph 9, wherein said gene expression level is a gene expression level of at least one of the genes of step (a).

11. Method of any of numbered paragraphs 8 to 10, wherein step (d) comprises applying a decision tree.

We identified a unique panel of genes combined into an algorithm for the here presented new prognostic test. The algorithm makes use of kinetic RT-PCR data from breast cancer patients and was trained on follow-up data for events like distant metastasis, local recurrence or death and data for non-events or long disease-free survival (healthy at last contact when seeing the treating physician).

The genes were selected of the following list of genes: ACTG1, CA12, CALM2, CCND1, CHPT1, CLEC2B, CTSB, CXCL13, DCN, DHRS2, EIF4B, ERBB2, ESR1, FBXO28, GABRP, GAPDH, H2AFZ, IGFBP3, IGHG1, IGKC, KCTD3, KIAA0101, KRT17, MLPH, MMP1, NAT1, NEK2, NR2F2, OAZ1, PCNA, PDLIM5, PGR, PPIA, PRC1, RACGAP1, RPL37A, SOX4, TOP2A, UBE2C and VEGF.

Different prognosis algorithms were built using these genes by selecting appropriate subsets of genes and combining their measurement values by mathematical functions. The function value is a real-valued risk score indicating the likelihoods of clinical outcomes; it can further be discriminated into two, three or more classes indicating patients to have low, intermediate or high risk. We also calculated thresholds for discrimination.

Table 1: List of Genes used in the method of the invention:

| List of Genes of algorithm P2e_Mz10 and P2e_lin: | | | |
|---|---|---|---|
| **Gene** | **Name** | **Process** | **Accession Number** |
| ESR1 | Estrogen Receptor | Hormone Receptor | NM_000125 |
| PGR | Progesteron Receptor | Hormone Receptor | NM_000926 |

(continued)

| List of Genes of algorithm P2e_Mz10 and P2e_lin: | | | |
|---|---|---|---|
| **Gene** | **Name** | **Process** | **Accession Number** |
| MLPH | Melanophilin | Hormone Receptor | NM_001042467 |
| TOP2A | Topoisomerase II alpha | Proliferation | NM_001067 |
| RACGAP1 | Rac GTPase activating Protein 1 | Proliferation | NM_001126103 |
| CHPT1 | Choline Phosphotransferase 1 | Proliferation | NM_020244 |
| MMP1 | Matrixmetallopeptidase | Invasion | NM 002421 |
| IGKC | Immuglobulin kappa constant | Immune System | NG_000834 |
| CXCL13 | Chemokine (C-X-C motif) Ligand 13 | Immune System | NM_006419 |
| CALM2 | Calmodulin 2 | Reference Genes | NM 001743 |
| PPIA | Peptidylprolyl Isomerase A | Reference Genes | NM_021130 |
| PAEP | Progestagen-associated Endometrial Protein | DNA Control | NM_001018049 |

Table 2: List of genes used in further algorithms according to the method of the invention List of Genes of further algorithms:

| Gene | Algorithms | | | | | | | Accession Number |
|---|---|---|---|---|---|---|---|---|
| | P1c | P2e | P2e c | P2e Mz10 | P7a | P7b | P7c | |
| | CorrDiff.9 | | | P2e Mz10 b | | | | |
| | | | | P2e lin | | | | |
| CALM2 | | | | | | | | NM_001743 |
| CHPT1 | | x | x | x | | | x | NM_020244 |
| CLEC2B | | | | | | | | NM_005127 |
| CXCL13 | x | x | x | x | x | x | x | NM_006419 |
| DHRS2 | | | | | | | | NM_005794 |
| ERBB2 | | | | | | | | NM_001005862 |
| ESR1 | x | x | x | x | | | | NM_000125 |
| FHL1 | | x | x | | | | | NM_001449 |
| GAPDH | | | | | | | | NM_002046 |
| IGHG1 | | | | | | | | NG_001019 |
| IGKC | x | x | x | x | x | x | x | NG_000834 |
| KCTD3 | | | | | | | | NM_016121 |
| MLPH | x | x | x | x | x | | | NM_001042467 |
| MMP1 | x | x | x | x | | x | x | NM_002421 |
| PGR | x | x | x | x | x | x | x | NM_000926 |
| PPIA | | | | | | | | NM_021130 |
| RACGAP1 | | x | x | x | x | x | x | NM_001126103 |

(continued)

| Gene | Algorithms | | | | | | | Accession Number |
|---|---|---|---|---|---|---|---|---|
| RPL37A | | | | | | | | NM_000998 |
| SOX4 | | x | | | | x | | NM_003107 |
| TOP2A | x | x | x | x | | | | NM_001067 |
| UBE2C | x | | | | x | x | x | NM_007019 |
| VEGF | | x | x | | | | x | NM_001025366 |
| # genes of interest | 8 | 12 | 11 | 9 | 7 | 6 | 8 | |

Example1: Algorithm P2e_Mz10

Algorithm P2e_Mz10 works as follows. Replicate measurements are summarized by averaging. Quality control is done by estimating the total RNA and DNA amounts. Variations in RNA amount are compensated by subtracting measurement values of housekeeper genes to yield so called delta CT values (difference in cycle threshold in quantitative PCR methods). Delta CT values are bounded to gene-dependent ranges to reduce the effect of measurement outliers. Biologically related genes were summarized into motives: ESR1, PGR and MLPH into motive "estrogen receptor", TOP2A and RACGAP1 into motive "proliferation" and IGKC and CXCL13 into motive "immune system". According to the RNA-based estrogen receptor motive and the progesteron receptor status gene cases were classified into three subtypes ER-, ER+/PR- and ER+/PR+ by a decision tree, partially fuzzy. For each tree node the risk score is estimated by a linear combination of selected genes and motives: immune system, proliferation, MMP1 and PGR for the ER- leaf, immune system, proliferation, MMP1 and PGR for the ER+/PR- leaf, and immune system, proliferation, MMP1 and CHPT1 for the ER+/PR+ leaf. Risk scores of leaves are balanced by mathematical transformation to yield a combined score characterizing all patients. Patients are discriminated into high and low risk by applying a threshold on the combined score. The threshold was chosen to achieve a sensitivity of about 90% and a specificity as high as possible on the prediction of distant metastases.

Technically, the test will rely on two core technologies: 1.) Isolation of total RNA from fresh or fixed tumor tissue and 2.) Kinetic RT-PCR of the isolated nucleic acids. Both technologies are available at SMS-DS and are currently developed for the market as a part of the Phoenix program. RNA isolation will employ the same silica-coated magnetic particles already planned for the first release of Phoenix products. The assay results will be linked together by a software algorithm computing the likely risk of getting metastasis as low, (intermediate) or high.

Most algorithms rely on many genes, to be measured by chip technology (> 70) or PCR-based (>15), and a complicated normalization of data (hundreds of housekeeping genes on chips) by not a less complicated algorithm that combines all data to a final score or risk prediction. MammaprintTM (70 genes and hundreds of normalization genes; OncotypeDXTM 16 genes and 5 normalization genes). We used a FFPE (formalin-fixed, paraffin-embedded) tumor sample collection of node-negative breast cancer patients with long-term follow-up data to prepare RNA and measure the amount of RNA of several breast cancer informative genes by quantitative RT-PCR. We identified algorithms that use fewer genes (8 or 9 genes of interest and only 1 or two reference or housekeeping genes). Denmark1 cohort: Death after recurrence within 5 years endpoint.

Transbig cohort: Distant metastasis within 10 years endpoint
Table 3 shows Area under the curve (AUC) of ROC curves (receiver operator curves) calculations for different algorithms at the working point (threshold between low and high risk) in the respective verification cohorts (Denmark1 or Transbig).

Table 3: AUC or ROC Curves for patient cohorts

**Denmark1 cohort: Death after recurrence within 5 years endpoint:**

| Algorithm | AUC of ROC curve |
|---|---|
| P1c | 0,77 |
| P2e | 0,61 |
| P2e_c | 0,81 |
| P2e_Mz10 | 0,76 |
| P2e_Mz10_b | 0,74 |

(continued)

**Denmark1 cohort: Death after recurrence within 5 years endpoint:**

| Algorithm | AUC of ROC curve |
|---|---|
| P2e_lin | 0,79 |
| P7a | 0,77 |
| P7b | 0,81 |
| P7c | 0,81 |

**Transbig cohort: Distant metastasis within 10 years endpoint**

| Algorithm | AUC of ROC curve |
|---|---|
| P1c | 0,72 |
| P2e | 0,71 |
| P2e_c | 0,69 |
| P2e_Mz10 | 0,71 |
| P2e_Mz10_b | 0,69 |
| P2e_lin | 0,73 |
| P7a | 0,71 |
| P7b | 0,72 |
| P7c | 0,73 |

Example 2: Detection of Gene Expression by RT PCR

Gene expression can be determined by a variety of methods, such as quantitative PCR, Microarray-based technologies and others.

In a representative example, quantitative reverse transcriptase PCR was performed according to the following protocol:

Primer/Probe Mix:

| 50 µl | 100 µM Stock Solution Forward Primer |
|---|---|
| 50 µl | 100 µM Stock Solution Reverse Primer |
| 25 µl | 100 µM Stock Solution Taq Man Probe bring to 1000 µl with water |
| 10 µl | Primer/Probe Mix (1:10) are lyophilized, 2.5 h RT |

RT-PCR Assay set-up for 1 well:

| 3.1 | µl Water |
|---|---|
| 5.2 | µl RT qPCR MasterMix (Invitrogen) with ROX dye |
| 0.5 | µl MgSO4 (to 5.5 mM final concentration) |
| 1 | µl Primer/Probe Mix dried |
| 0.2 | µl RT/Taq Mx (-RT: 0.08 µL Taq) |
| 1 | µl RNA (1:2) |

Thermal Profile:

RT step

| 50 °C | 30 Min * |
|---|---|
| 8 °C | ca. 20 Min * |
| 95 °C | 2 Min |

PCR cycles (repeated for 40 cycles)

| 95 °C | 15 Sec. |
|---|---|
| 60 °C | 30 Sec. |

Gene expression can be determined by known quantitative PCR methods and devices, such as TagMan, Lightcycler and the like. It can then be expressed e.g. as cycle threshold value (CT value) .

Example 4: matlab Scripts for algorithms of the invention

The following is a Matlab script to calculate from raw Ct value the risk prediction of a patient containing examples of some of the algorithms used in the invention (Matlab R2007b, Version 7.5.0.342, © by The MathWorks Inc.). User-defined comments are contained in lines preceded by the "%" symbol. These comments are overread by the program and are for the purpose of informing the user/reader of the script only. Command lines are not preceded by the "%" symbol:

```
function risk = predict(e, type) ¶
 % input "e": gene expression values of patients. Variable "e" is of type¶
 % struct, each field is a numeric vector of expression values of the¶
 % patients. The field name corresponds to the gene name. Expression¶
 % values are pre-processed delta-CT values. ¶
 % input "type": name of the algorithm (string) ¶
 % output risk: vector of risk scores for the patients. The higher the
 score ¶
 % the higher the estimated probability for a metastasis or desease-¶
 % related death to occur within 5 or 10 years after surgery. Negative¶
 % risk scores are called "low risk", positive risk score are called
 "high¶
 % risk". ¶
 switch type¶
   case 'Plc'¶
      % adjust values for platform¶
       CXCL13 = (e.CXCL13 -11.752821) / 1.019727 + 8.779238;¶
       ESR1 = (e.ESR1 -15.626214) / 1.178223 + 10.500000;¶
       IGKC = (e.IGKC -11.752725) / 1.731738 + 11.569842;¶
      MLPH = (e.MLPH -14.185453) / 2.039551 + 11.000000;¶
      MMP1 = (e.MMP1 - 9.484186) / 0.987988 + 6.853865;¶
       PGR = (e.PGR -13.350160) / 0.953809 + 6.000000;¶
       TOP2A = (e.TOP2A -13.027047) / 1.300098 + 9.174689;¶
      UBE2C = (e.UBE2C -14.056418) / 1.160254 + 9.853476;¶
 ¶
       % prediction of subtype¶
      srNoise = 0.5;¶
      info.srStatusConti = 2 * logit((ESR1-10.5)/srNoise) + logit((PGR-
6)/srNoise) + logit((MLPH-11)/srNoise);¶
      info.srStatus = (info.srStatusConti >= 2) + 0;¶
      prNoise = 1;¶
      info.prStatus = logit((PGR-6)/prNoise);¶
      info.wgt0 = 1 - info.srStatus;¶
      info.wgt1 = info.srStatus .* (1-info.prStatus);¶
      info.wgt2 = info.srStatus .* info.prStatus;¶
 ¶
      % risks of subtypes¶
      info.risk0 = (logit((CXCL13-10.194199)*-0.307769) + ...¶
                   logit((IGKC-12.314798)*-0.382648) + . . .¶
                   logit((MLPH-10.842093)*-0.218234) + . . .¶
                   logit((MMP1-8.201517)*0.157167) + . . . ¶
                   logit((ESR1-9.031409)*-0.285311) -2.623903) *
2.806133;¶
      info.risk1 = (logit((TOP2A-8.820398)*0.697681) + ...¶
                   logit((UBE2C-9.784955)*1.123699) + ...¶
                   logit((PGR-5.387180)*-0.328050) -1.616721) *
2.474979;¶
      info.risk2 = (logit((CXCL13-4.989277)*-0.142064) + . . .¶
                   logit((IGKC-8.854017)*-0.232467) + ...¶
                   logit((MMP1-9.971173)*0.127538) -1.321320) *
3.267279;¶
 ¶
      % final risk¶
```

```
      risk = info.risk0 .* info.wgt0 + info.risk1 .* info.wgt1 +
info.risk2 .* info.wgt2 + 0.8;¶
¶
  case 'P2e'¶
      % adjust values for platform¶
      ESR1 = (e.ESR1 -15.652953) / 1.163477 + 10.500000;¶
      MLPH = (e.MLPH -14.185453) / 2.037305 + 11.000000;¶
      PGR = (e.PGR -13.350160) / 0.957324 + 6.000000;¶
¶
      % prediction of subtype¶
      srNoise = 0.5;¶
      info.srStatusConti = 2 * logit((ESR1-10.5)/srNoise) + logit((PGR-
6)/srNoise) + logit((MLPH-11)/srNoise);¶
      info.srStatus = (info.srStatusConti >= 2) + 0;¶
      prNoise = 1;¶
      info.prStatus = logit((PGR-6)/prNoise);¶
      info.wgt0 = 1 - info.srStatus;¶
      info.wgt1 = info.srStatus .* (1-info.prStatus);¶
      info.wgt2 = info.srStatus .* info.prStatus;¶
¶
      % motives¶
      immune = e.IGKC + e.CXCL13;¶
    prolif = 1.5 * e.RACGAP1 + e.TOP2A;¶
       ¶
      % risks of subtypes¶
      info.risk0 = . . .¶
                +-0.0649147*immune . . .¶
                + 0.2972054*e.FHL1 . . .¶
                + 0.0619860*prolif . . .¶
                + 0.0283435*e.MMP1 . . .¶
                + 0.0596162*e.VEGF . . .¶
                +-0.0403737*e.MLPH . . .¶
                +-4.1421322;¶
      info.risk1 = . . .¶
                +-0.0329128*e.FHL1 . . .¶
                + 0.1052475*prolif . . .¶
                + 0.0293242*e.MMP1 . . .¶
                +-0.1035659*e.PGR . . .¶
                 + 0.0738236*e.SOX4 ...¶
                 +-3.1319335;¶
      info.risk2 = ...¶
                +-0.0363946*immune . . .¶
                + 0.0717352*prolif . . .¶
                +-0.1373369*e.CHPT1 . . .¶
                + 0.0840428*e.SOX4 . . .¶
                + 0.0157587*e.MMP1 . . .¶
                +-0.9378916;¶
¶
      % final risk¶
      risk = info.risk0 .* info.wgt0 + info.risk1 .* info.wgt1 +
info.risk2 .* info.wgt2 + 0.6;¶
    ¶
  case 'P2e_c'¶
      % adjust values for platform¶
      ESR1 = (e.ESR1 -15.652953) / 1.163477 + 10.500000;¶
      MLPH = (e.MLPH -14.185453) / 2.037305 + 11.000000;¶
      PGR = (e.PGR -13.350160) / 0.957324 + 6.000000;¶
¶
      % prediction of subtype¶
      srNoise = 0.5;¶
      info.srStatusConti = 2 * logit((ESR1-10.5)/srNoise) + logit((PGR-
6)/srNoise) + logit((MLPH-11)/srNoise);¶
      info.srStatus = (info.srStatusConti >= 2) + 0;¶
```

```
        prNoise = 1;¶
        info.prStatus = logit((PGR-6)/prNoise);¶
        info.wgt0 = 1 - info.srStatus;¶
        info.wgt1 = info.srStatus .* (1-info.prStatus);¶
        info.wgt2 = info.srStatus .* info.prStatus;¶
¶
        % motives¶
        immune = 0.5 * e.IGKC + 0.5 * e.CXCL13;¶
        prolif = 0.6 * e.RACGAP1 + 0.4 * e.TOP2A;¶
        ¶
        % risks of subtypes¶
        info.risk0 = . . .¶
                    +-0.1283655*immune . . .¶
                     + 0.3106840*e.FHL1 . . .¶
                     + 0.0319581*e.MMP1 . . .¶
                     + 0.2304728*prolif . . .¶
                     + 0.0711659*e.VEGF . . .¶
                     + 0.0123868*e.ESR1 . . .¶
                     +-6.1644527 + 1;¶
        info.risk1 = . . .¶
                     + 0.3018777*prolif . . .¶
                    +-0.0992731*e.PGR . . .¶
                     + 0.0351513*e.MMP1 . . .¶
                    +-0.0302850*e.FHL1 . . .¶
                    +-2.5403380;¶
        info.risk2 = . . .¶
                     + 0.1989859*prolif . . .¶
                    +-0.1252159*e.CHPT1 . . .¶
                    +-0.0808729*immune . . .¶
                     + 0.0227976*e.MMP1 . . .¶
                     + 0.0433237;¶
¶
        % final risk¶
        risk = info.risk0 .* info.wgt0 + info.risk1 .* info.wgt1 +
info.risk2 .* info.wgt2 + 0.3;¶
¶
  case 'P2e_Mz10'¶
        % adjust values for platform¶
        ESR1 = (e.ESR1 -15.652953) / 1.163477 + 10.500000;¶
        MLPH = (e.MLPH -14.185453) / 2.037305 + 11.000000;¶
        PGR = (e.PGR -13.350160) / 0.957324 + 6.000000;¶
¶
        % prediction of subtype¶
        srNoise = 0.5;¶
        info.srStatusConti = 2 * logit((ESR1-11)/srNoise) + logit((PGR-
6)/srNoise) + logit((MLPH-11)/srNoise);¶
        info.srStatus = (info.srStatusConti >= 2) + 0;¶
        prNoise = 1;¶
        info.prStatus = logit((PGR-6)/prNoise);¶
        info.wgt0 = 1 - info.srStatus;¶
        info.wgt1 = info.srStatus .* (1-info.prStatus);¶
        info.wgt2 = info.srStatus .* info-prstatus;¶
        ¶
        % motives¶
        immune = 0.5 * e.IGKC + 0.5 * e.CXCL13;¶
        prolif = 0.6 * e.RACGAP1 + 0.4 * e.TOP2A;¶
¶
        % risks of subtypes¶
        info.risk0 = +-0.1695553*immune + 0.2442442*prolif +
0.0576508*e.MMP1 +-0.0329610*e.PGR +-1.2666276;¶
        info.risk1 = +-0.1014611*immune + 0.1520673*prolif +
0.0127294*e.MMP1 +-0.0724982*e.PGR + 0.0307697;¶
        info.risk2 = +-0.1209503*immune + 0.0491344*prolif +
```

```
0.0749897*e.MMP1 +-0.0602048*e.CHPT1 + 0.8781799;¶
    ¶
    % final risk¶
    risk = info.risk0 .* info.wgt0 + info.risk1 .* info.wgt1 +
info.risk2 .* info.wgt2 + 0.25;¶
¶
  case 'P2e_Mz10_b'¶
    % adjust values for platform¶
    ESR1 = (e.ESR1 -15.652953) / 1.163477 + 10.500000;¶
    MLPH = (e.MLPH -14.185453) / 2.037305 + 11.000000;¶
    PGR = (e.PGR -13.350160) / 0.957324 + 6.000000;¶
¶
    % prediction of subtype¶
    srNoise = 0.5;¶
    info.srStatusConti = 2 * logit((ESR1-11)/srNoise) + logit((PGR-
6)/srNoise) + logit((MLPH-l1)/srNoise);¶
    info.srStatus = (info.srStatusConti >= 2) + 0;¶
    prNoise = 1;¶
    info.prStatus = logit((PGR-6)/prNoise);¶
    info.wgt0 = 1 - info.srstatus;¶
    info.wgt1 = info.srStatus .* (1-info.prStatus);¶
    info.wgt2 = info.srStatus .* info.prStatus;¶
    ¶
    % motives¶
    immune = 0.5 * e.IGKC + 0.5 * e.CXCL13;¶
    prolif = 0.6 * e.RACGAP1 + 0.4 * e.TOP2A;¶
¶
    % risks of subtypes¶
    info.risk0 = +-0.1310102*immune + 0.1845093*prolif +
0.1511828*e.CHPT1 +-0.1024023*e.PGR +-2.0607350;¶
    info.risk1 = +-0.0951339*immune + 0.1271194*prolif +-
0.1865775*e.CHPT1 +-0.0365784*e.PGR + 2.9353027;¶
    info.risk2 = +-0.1209503*immune + 0.0491344*prolif +-
0.0602048*e.CHPT1 + 0.0749897*e.MMP1 + 0.8781799;¶
    ¶
    % final risk¶
    risk = info.risk0 .* info.wgt0 + info.risk1 .* info.wgt1 +
info.risk2 .* info.wgt2 + 0.3;¶
¶
  case 'P2e_lin' ¶
    % motives¶
    estrogen = 0.5 * e.ESR1 + 0.3 * e.PGR + 0.2 * e.MLPH;¶
    immune = 0.5 * e.IGKC + 0.5 * e.CXCL13;¶
    prolif = 0.6 * e.RACGAP1 + 0.4 * e.TOP2A;¶
¶
    % final risk¶
    risk = +-0.0733386*estrogen . . .¶
        +-0.1346660*immune . . .¶
        + 0.1468378*prolif . . .¶
        + 0.0397999*e.MMP1 . . .¶
        +-0.0151972*e.CHPT1 . . .¶
        + 0.6615265 . . .¶
        + 0.25;¶
¶
  case 'P7a' ¶
    % motives¶
    prolif = 0.6 * e.RACGAP1 + 0.4 * e.UBE2C;¶
    immune = 0.5 * e.IGKC + 0.5 * e.CXCL13;¶
    estrogen = 0.5 * e.MLPH + 0.5 * e.PGR;¶
¶
    % final risk¶
    risk = +0.2944 * prolif . . . ¶
        -0.2511 * immune . . . ¶
```

```
                -0.2271 * estrogen . . .¶
                +0.3865 * e.SOX4 ... ¶
                -3.3;¶
        ¶
    case 'P7b' ¶
        % motives¶
        prolif = 0.6 * e.RACGAP1 + 0.4 * e.UBE2C;¶
        immune = 0.5 * e.IGKC + 0.5 * e.CXCL13;¶
    ¶
        % final risk¶
        risk = +0.4127 * prolif . . .¶
                -0.1921 * immune . . .¶
                -0.1159 * e.PGR . . . ¶
                +0.0876 * e.MMP1 . . .¶
                -1.95;¶
        ¶
    case 'P7c' ¶
        % motives¶
        prolif = 0.6 * e.RACGAP1 + 0.4 * e.UBE2C;¶
        immune = 0.5 * e.IGKC + 0.5 * e.CXCL13;¶
    ¶
        % final risk¶
        risk = +0.4084 * prolif . . . ¶
                -0.1891 * immune . . . ¶
                -0.1017 * e.PGR . . . ¶
                +0.0775 * e.MMP1 . . . ¶
                +0.0693 * e.VEGF . . . ¶
                -0.0668 * e.CHPT1 . . .¶
                -1.95;¶
    ¶
    otherwise¶
        error('unknown algorithm');¶
end¶
end¶
¶
¶
¶
function y = logit(x)¶
y = 1./(1 + exp(-x)); ¶
end¶
¶
¶
¶
% end of file¶
```

The following is a Matlab script containing a further example of an algorithm used in the invention (Matlab R2007b, Version 7.5.0.342, © by The MathWorks Inc.). User-defined comments are contained in lines preceded by the "%" symbol. These comments are overread by the program and are for the purpose iof informing the user/reader of the script only. Command lines are not preceded by the "%" symbol:

```
function risk = predict(e)¶
 % input "e": gene expression values of patients. Variable "e" is of type¶
 % struct, each field is a numeric vector of expression values of the¶
 % patients. The field name corresponds to the gene name. Expression¶
 % values are pre-processed delta-CT values.¶
 % output risk: vector of risk scores for the patients. The higher the
 score¶
 % the higher the estimated probability for a metastasis or disease-¶
 % related death to occur within 5 or 10 years after surgery. Negative¶
 % risk scores are called "low risk", positive risk score are called
 "high¶
 % risk".¶
```

```
expr = [20 * ones(size(e.CXCL13)), ... % Housekeeper HKM¶
  e.CXCL13, e.ESR1, e.IGKC, e.MLPH, e.MMP1, e.PGR, e.TOP2A, e.UBE2C];¶

m = [ . . .¶
  20, 20; ...¶
  11.817, 11.1456; ...¶
  17.1194, 16.7523; ...¶
  11.6005, 10.046; ...¶
  16.6452, 16.1309; ...¶
  9.54657, 10.9477; ...¶
  13.181, 12.0208; . . .¶
  12.9811, 13.811; . . .¶
  14.1037, 14.708];¶
risk = corr(expr', m(:, 2)) - corr(expr', m(:, 1)) + 0.08;¶
end¶



% end of file¶
```

The following is a Matlab script file which contains an implementation of the prognosis algorithm including the whole data pre-processing of raw CT values (Matlab R2007b, Version 7.5.0.342, © by The MathWorks Inc.. The pre-processed delta CT values may be directly used in the above described algorithms:

```
function FromRawCTtoRisk¶

%%%%%%%%%%%%%%%%%%%%%%%%%%%%%%%%%%%%%%%%%%%%%%%%%%%%%%%%%%%%%%%%%%%%
%%¶
% This is the main program. It reads raw data and processes it through¶
% various sub-functions to generate preprocessed delta CT values,¶
% preprocessed clinical outcome and risks for the P2e_Mz10 algorithm.¶



% Load data to apply to algorithm¶
load PatientData.mat; % creates variables "data" and "datGenes"¶
% Variable "datGenes" is of type vector of strings and contains the names
of ¶
% genes measured.¶
% Variable "data" is of type struct. Each field is a vector whose length
¶
% equals the number of patients. Field "bc" contains the patient
identifiers.¶
% For each gene measured a field exists containing the measured raw CT
values ¶
% (averages over replicate measurements). Several additional fields with
¶
% self-explaining names contain clinical and follow-up information.¶

% Remove patients according to quality criteria.¶
data = selectValidPatients(data, datGenes);¶
% Variable "data" is of type struct ¶

% Calculate delta CT values, remove clinical information other than¶
% endpoint.1
[expr, patients] = calculateDeltaCTvalues(data);l
% Variable "expr" is of type struct¶
% Variable "patients" is of type struct ¶

% At this point the cohort-dependent preprocessing is finished. We now ¶
% could train some new algorithm using the outcome coded in variable ¶
```

```
% "patients". ¶
¶
% Reduce the effect of outliers. Set "Undetermined" measurements (minus ¶
% infinity here) to the lower bound. ¶
expr = applyBounds(expr); ¶
% Variable "expr" is of type struct ¶
¶
% Calculate risks according to algorithm P2e_Mz10 ¶
risk = predict_P2e_Mz10 (expr) ; ¶
% Variable "risk" is of type vector¶
¶
end¶
% end of main function¶
¶
¶
¶
function data = selectValidPatients (data, datGenes) ¶
% raw CT value of HKM must be below 32 to ensure enough RNA¶
HKM_MAX = 32;¶
% DNA is assumed to be absent if the raw CT value of PAEP is above 35¶
PAEP_MIN = 35;¶
% raw CT value of any bispecific gene must be below PAEP minus 3 to
ensure¶
% measurement of RNA not DNA. If DNA is absent (PEAP > 35) we assume
% measuring RNA regardless of the genes value.¶
PAEP_DISTANCE = 3;¶
% the set of gene specific to RNA as well as DNA¶
BISPECIFIC_GENES = {'CALM2', 'CLEC2B', 'ERBB2', 'HKM', 'KCTD3', 'PGR'
'PPIA', 'RPL37A', 'UBE2C'}';¶
¶
%%% check whether a patient is valid for different reasons¶
% memory initialization, set all patients to be valid for all reasons¶
validUndet = ones (size (data.bc));¶
validPaep = ones (size(data.bc));¶
validHKM = ones (size (data.bc)) ; ¶
% end of initialization¶
% process all patients separately¶
for i = 1:length(data.bc)¶
  % test RNA yield¶
  if data.HKM (i) > HKM_MAX¶
    validHKM(i) = 0;¶
  end¶
  ¶
  % test all genes to be NaN. A gene is NaN for some patient iff all¶
  % triplicates have been NaN (=empty cell in Excel).¶
  for k = 1:length (datGenes) ¶
    if isnan (data. (datGenes {k} ) (i) )¶
        validUndet (i) = 0;¶
    end¶
  end¶
¶
  % test all bispecific genes to measure RNA not DNA¶
  for k = BISPECIFIC_GENES'¶
    % get raw CT values of PEAP and the gene¶
    paepValue = nanmean data.PAEP(i) ) ; ¶
    geneValue = data.(k{1}) (i);¶
    % if the gene is nearby PAEP and PAEP is detected, then
invalidate¶
     % patient ¶
     if geneValue > paepValue - PAEP_DISTANCE && paepValue <=
PAEP_MIN¶
         validPaep (i) = 0 ; ¶
     end¶
```

```
    end¶
  end¶
  ¶
  % remove patients for any reason¶
  valid = validPaep & validUndet & validHKM;¶
  for f = fields (data) ' ¶
    data.(f{1}) = data. (f{1}) (valid) ; ¶
  end¶
  ¶
  % PEAP is no longer needed, remove this information¶
  data = rmfield(data, 'PAEP');¶
  end¶
  ¶
  ¶
  ¶
  function [e, patients] = calculateDeltaCTvalues (data) ¶
  % these OFFSETS compensate changes in SOPs¶
  OFFSETS = { . . .¶
    'CALM2', -0.982189; . . .¶
    'CHPT1', -0.815981; . . .¶
    'CLEC2B', 0.889707; . . .¶
    'CXCL13', 0.118816; . . .¶
    'DHRS2', -0.0541808; . . .¶
    'ERBB2', -0.317573; . . .¶
    'ESR1', 0.366009; . . .¶
    'GAPDH', -0.751933; . . .¶
    'IGHG1', -0.109166; . . .¶
    'IGKC', -0.243044; . . .¶
    'KCTD3', -0.166892; . . .¶
    'MLPH', -0.26247; . . .¶
    'MMP1', -0.0922985; . . .¶
    'PGR', -0.109736; . . .¶
    'PPIA', -0.495741; . . .¶
    'RACGAP1', -0.887749; . . .¶
    'RPL37A', -0.906199; . . .¶
    'SOX4', -0.190544; . . .¶
    'TOP2A', -0.166589; . . .¶
    'UBE2C' , -0.0249995; . . .¶
    'VEGF', -0.85129};¶
  % manually added¶
  OFFSETS (end+1, :) = {'FHL1', 10.3773 - 14.2705};¶
  ¶
  % get the set of genes (except HKM which is a housekeeper and can not be¶
  % adjusted)¶
  f = fields (data) ;¶
  f = f(strcmp(f, upper(f)) & -strcmp(f, 'HKM'));¶
  % process all genes: convert the value of 40 to infinity. Note: the
  value¶
  % 40 occurs iff all triplicates were "Undetermined".¶
  for i = 1:length (f) ¶
    d = data. (f{i});¶
    d(d == 40) = inf;¶
    data.(f{i}) = d;¶
  end¶
  ¶
  %%% calculate delta CT values¶
  % use the mean of CALM2 and PPIA as housekeeper value¶
  house = 0.5 * (data.CALM2 + data.PPIA) ; ¶
  % calculate delta CT values for all genes¶
  for i = 1:length (f) ¶
    % our definition of delta CT values¶
    deltaCT = 20 - data.(f{i}) + house;¶
    % search for offset¶
```

```
    idx = find(strcmp(f{i}, OFFSETS(:, 1)));¶
    if length(idx) ~= 1¶
        error('no offset defined') ; ¶
  end¶
  % Add offset to normalize to the scale needed by the algorithms¶
 e.(f{i}) = deltaCT + OFFSETS {idx, 2 } ; ¶
end¶
¶
% copy patient names¶
patients.names = data.bc;¶
% our endpoint is death after recurrence here. Variable "events" contains
¶
% the corresponding flag.¶
events = (data.death & strcmp(data.recurrence, 'yes')) ;¶
% check consistency of data¶
if any(data.deathTime(events) < data.recurrenceTime(idx)) ¶
    error('death before recurrence');¶
end¶
¶
%%% store endpoint information. NOTE: the fields "patients.metastasis"
and ¶
% "patients.mfi" does not contain the metastasis information here, they
are¶
% just part of the software interface and contain the endpoint flag and ¶
% time.¶
%¶
% store endpoint flag¶
patients.metastasis = events + 0;¶
% store endpoint time for events¶
patients.mfi = data.deathTime;¶
% store censoring time for non-events in the same variable¶
patients.mfi(~events) = data.followUp(~events);¶
¶
% filter patients: remove all patients not in stratum 1¶
valid = (data.stratum == 1);¶
for f = fields (patients) ' ¶
    patients.(f{1}) = patients.(f{1}) (valid);¶
end¶
for f = fields(e) '¶
    e. (f{1}) = e. (f{1}) (valid);¶
end¶
end¶
¶
¶
¶
function e = applyBounds(e) ¶
% these BOUNDS are designed to reduce the effect of measurement outliers¶
% and to set the "Undetermined" values to some well-defined, ¶
% housekeeper-independent value.¶
BOUNDS = { . . .¶
  'CALM2', 17.7768, 19.3293; . . .¶
  'CHPT1', 13.8922, 19.3063; . . .¶
  'CLEC2B', 9.31008, 13.891; . . .¶
  'CXCL13', 5.09159, 19.3774; . . .¶
  'DHRS2', 5.9009, 20.4205; . . .¶
  'ERBB2', 13.9414, 20.9202; . . .¶
  'ESR1', 11.295, 20.7089; . . .¶
  'GAPDH', 16.7778, 19.4194; . . .¶
  'IGHG1', 8.30258, 20.1163; . . .¶
  'IGKC', 7.26622, 17.2448; . . .¶
  'KCTD3', 13.0252, 17.104; . . .¶
  'MLPH', 11.3369, 20.0319; . . .¶
  'MMP1', 5.47869, 15.2577; . . .¶
```

```
          'PGR', 7.19061, 17.5858; . . .¶
          'PPIA', 19.369, 20.7453; . . .¶
          'RACGAP1', 10.4169, 14.4016; . . .¶
          'RPL37A', 19.3326, 21.9422; . . .¶
          'SOX4', 15.1768, 18.4415; . . .¶
          'TOP2A', 9.70211, 16.3468; . . .¶
          'UBE2C', 11.6724, 16.5079; . . .¶
          'VEGF', 13.0577, 16.6318};¶
¶
% process all genes¶
f = fields(e);¶
for i = 1:length(f)¶
  % variable "d" contains delta CT values for all patients¶
  d = e.(f{i});¶
  % search for applicable bounds¶
  idx = find(strcmp(BOUNDS(:, 1), f {i}));¶
  switch length(idx)
      case 0¶
          % no bounds defined, set bounds to extremal values without¶
          % considering "Undetermined" values.¶
          % NOTE: This happens for FHL1 only, which is not used by¶
          % algorithm P2e_Mz10.¶
          lowerBound = min(d(d > -inf));¶
          upperBound = max(d(d < inf));¶
        case 1¶
          lowerBound = BOUNDS {idx, 2};¶
          upperBound = BOUNDS {idx, 3};¶
        otherwise¶
          error('multiple bounds');¶
  end¶
  % apply bounds: set everything to "lowerBound" which is below¶
  d(d < lowerBound) = lowerbound;¶
  % set everything to "upperBound" which is above¶
  d(d > upperBound) = upperBound;¶
  % store bounded data¶
  e.(f{i}) = d;¶
end¶
end¶
¶
¶
¶
function risk = predict_P2e-Mz10 (e) ¶
% adjust values for platform¶
ESR1 = (e.ESR1 -15.652953) / 1.163477 + 10.500000;¶
MLPH = (e.MLPH -14.185453) / 2.037305 + 11.000000;¶
PGR = (e.PGR -13.350160) / 0.957324 + 6.000000;¶
¶
% prediction of subtype¶
srNoise = 0.5;¶
info.srStatusConti = 2 * logit ((ESR1-11)/srNoise) + logit((PGR-
6) / srNoise) + logit ((MLPH-11) / srNoise);¶
info.srStatus = (info.srStatusConti >= 2) + 0;¶
prNoise = 1;¶
info.prStatus = logit ((PGR-6)/prNoise);¶
info.wgt0 = 1 - info.srStatus;¶
info.wgt1 = info.srStatus .* (1-info.prStatus);¶
info.wgt2 = info.srStatus .* info.prStatus;¶
¶
% motives¶
immune = 0.5 * e.IGKC + 0.5 * e.CXCL13;¶
prolif = 0.6 * e.RACGAP1 + 0.4 * e.TOP2A;¶
¶
% risks of subtypes¶
```

```
info.risk0 = +-0.1695553*immune + 0.2442442*prolif + 0.0576508*e.MMP1 +-
0.0329610*e.PGR +-1.2666276;¶
info.risk1 = +-0.1014611*immune + 0.1520673*prolif + 0.0127294*e.MMP1 +-
0.0724982*e.PGR + 0.0307697;¶
info.risk2 = +-0.1209503*immune + 0.0491344*prolif + 0.0749897*e.MMP1 +-
0.0602048*e.CHPT1 + 0.8781799;¶
¶
% final risk¶
risk = info.risk0 .* info.wgt0 + info.risk1 .* info.wgt1 + info.risk2 .*
info.wgt2 + 0.4;¶
end¶
¶
¶
¶
function y = logit (x)¶
y = 1./(1 + exp(-x)); ¶
end¶
¶
¶
% end of file¶
```

Example: CorrDiff.9:

As mentioned in Table 2, CorrDiff9 uses the same genes as the algorithm P1c (CXCL13, ESR1, IGKC, MLPH, MMP1, PGR, TOP2A, and UBE2C) and the housekeeping gene mixture, HKM, a mixture of 1, 2, 3 or 4 of the following genes: PPIA, OAZ1, GAPDH, RPL37A.

As in the above examples, we choose the Pearson correlation coefficient as a measure of similarity which has values between (and including) -1 and 1.

Patients are divided into two classes, e.g. patients that develop a distant metastasis within a given time frame of e.g. 5 years (denoted by "HR" as in "High Risk"), and those who do not develop a metastasis within this time frame (denoted consequently by "LR" as in "Low Risk").

From CT measurement values of these genes we calculate delta CT values according to the following definition:

$$dCT(gene) = 20 - CT(gene) + CT(HKM),$$

where "gene" is one of HKM, CXCL13, ESR1, IGKC, MLPH, MMP1, PGR, TOP2A, and UBE2C. We define the expression profile vector for sample $i$ as

$$P_i = (20, \; dCT(CXCL13), \; dCT(ESR1), \; dCT(IGKC), \; dCT(MLPH),$$
$$dCT(MMP1), \; dCT(PGR), \; dCT(TOP2A), \; dCT(UBE2C))'$$

From the training data mean expression profiles were calculated; we found

$$P_{ref,control} = (20; \; 11.82; \; 17.12; \; 11.60; \; 16.65;$$
$$9.55; \; 13.18; \; 12.98; \; 14.10)'$$

$$P_{ref,case} = (20; \; 11.15; \; 16.75; \; 10.05; \; 16.13;$$
$$10.95; \; 12.02; \; 13.81; \; 14.71)'$$

Risk assessment of an unknown patient sample i is calculated as

$$diff(P_i) = corr(P_i, P_{ref,case}) - corr(P_i, P_{ref,control}) ,$$

where *diff(P_i)* is the continuous-valued sample risk score and *corr* is the Pearson correlation coefficient. The higher *diff (P_i)* the higher the predicted risk to develop a metastasis within e.g. 5 years. Since the *corr* is invariant under linear transformation of one of its parameters *diff(P_i)* is unchanged if we change the definition of the expression profile vector to (non-delta) CT values:

$$P_i = (\texttt{CT(HKM), CT(CXCL13), CT(ESR1), CT(IGKC), CT(MLPH),}$$
$$\texttt{CT(MMP1), CT(PGR), CT(TOP2A), CT(UBE2C))'}$$

The risk classification is done by comparing the risk score by some threshold. As an example threshold -0.08 yields to a sensitivity of about 90% in validation data sets:

$$prediction(P_i) = \begin{cases} case & if \quad diff(P_i) > -0.08 \\ control & otherwise \end{cases}$$

Example of P2e_Mz10; testing of two different endpoints:

We tested the performance of P2e_Mz10 on two different endpoints: one is the prediction of metastasis within 5 years after surgery of the primary breast tumor, the other is the prediction overall survival within 10 years after surgery of the primary breast tumor. 141 patient samples were tested from a Danish cohort of patient ages below 60 years.
The area under the ROC curve (AUC) is given for the two endpoints distant metastasis within 5 years and overall survival within 10 years.

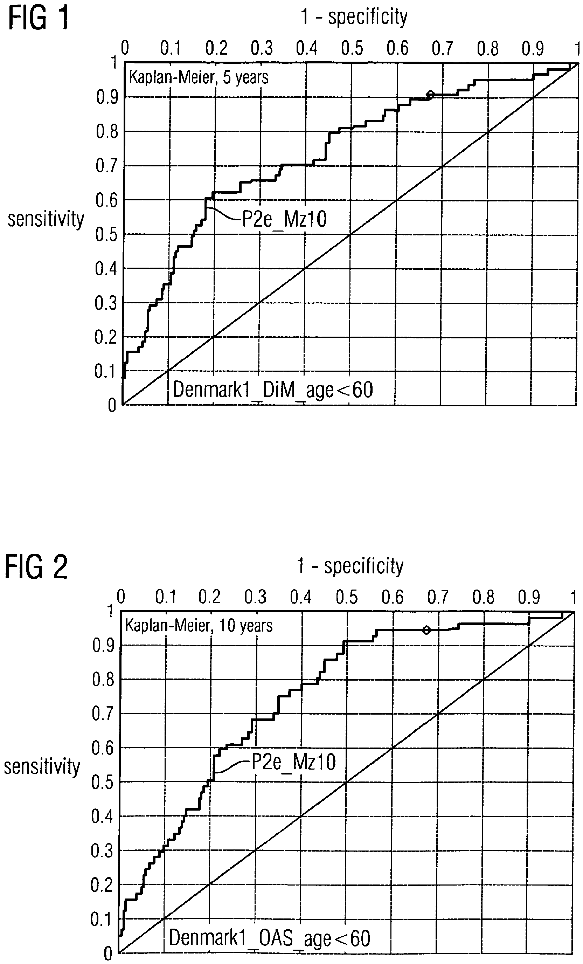

Fig. 1 shows the AUC of the ROC curve of algorithm P2e_Mz10 for the endpoint "Distant Metastasis within 5 years".
Sensitivity and specificity are 91% / 32% respectively, AUC: 0.74.
Fig. 2 shows the AUC of the ROC curve of algorithm P2e_Mz10 for the endpoint "Overall survival within 10 years"
Sensitivity and specificity are 95% / 33% respectively, AUC: 0.76
We found that the prediction of overall survival within 10 years is slightly better than the prediction of distant metastasis within 5 years although this difference is not significant.

Examples of highly correlated genes:

It is known that the expression of various genes correlate strongly. Therefore single or multiple genes used in the method of the invention may be replaced by other correlating genes. The following tables 4-12 give examples of correlating genes for each gene used in the above described methods, which may be used to replace single or multiple gene. The top line in each of the following tables contains the primary gene of interest, in the lines below are listed correlated genes, which may be used to replace the primary gene of interest in the above described methods.

Table 4 Examples of correlating genes

| RPL37A | GAPDH | ACTG1 | CALM2 |
|---|---|---|---|
| RPL38 | ENO1 | EEF1A1 | RPL41 |
| -- | PGK1 | RPS3A | EEF1A1 |
| EEF1 D | HSPA8 | RPL37A | RPS10 |
| RPLP2 | ACTB | RPLP0 | RPS27 |
| RPS10 | HSPCB | RPS23 | RPL37A |
| XTP2 | STIP1 | RPS28 | RPL39 |
| FKSG49 | ZNF207 | ACTB | ACTB |

(continued)

| RPL37A | GAPDH | ACTG1 | CALM2 |
|--------|-------|-------|-------|
| RPS11 | PSMC3 | RPL23A | RPLP0 |
| ENO1 | MSH6 | RPL7 | RPS3A |
| INHBC | TKT | RPL39 | RPS2 /// LOC91561 /// LOC148430 /// LOC286444 /// LOC400963 /// LOC440589 |
| RPL14 | PSAP | LOC389223 /// LOC440595 | PPIA |
| ATP6V0E | RAN | TPT1 | RPL3 |
| OPHN1 | GDI2 | RPL41 | RPS18 |
| JTV1 | WDR1 | HUWE1 | RPS2 |
| E2F4 | ILF2 | RPL3 | RPS12 |
| ATP6V1D | ABCF2 | RPL13A | ACTG1 |
| EIF5B | USP4 | RPS4X | RPL23A |
| CTAGE1 | HNRPC | RPS18 | RPL13A |
| NUCKS | MAPRE1 | RPS10 | MUC8 |
| TRA1 | C7orf28A /// C7orf28B | RPS17 | RPLP1 |

Table 5 Examples of correlating genes

| OAZ1 | PPIA | CLEC2B | CXCL13 |
|------|------|--------|--------|
| C19orf10 | K-ALPHA-1 | LY96 | TRBV19 /// TRBC1 |
| MED12 | ACTG1 | WASPIP | CD2 |
| AP2S1 | ACTB | DCN | CD52 |
| LOC222070 | RPS2 | SERPING1 | TNFRSF7 |
| CTGLF1 /// LOC399753 /// FLJ00312 /// CTGLF2 | RPL23A | C1S | CD3D |
| RAB1A | RPL39 | SERPINF1 | LCK |
| ARPC4 | RPL37A | PTGER4 | MS4A1 |
| ARFRP1 | GAPDH | CUGBP2 | CD48 |
| NUP214 | CHCHD2 | KCTD12 | SELL |
| POLR2E | RPS10 | EVI2A | IGHM |
| C2orf25 | RPL13A | HLA-E | POU2AF1 |
| UBE2D3 | TUBA6 | AXL | TRBV21-1 /// TRBV19 /// TRBV5-4 /// TRBV3-1 /// TRBC1 |
| ATP6V0E | RPLP0 | C1R | TRAC |
| XKR8 | RPL30 | CFH /// CFHL1 | CCL5 |
| LOC401210 | GNAS | PTPRC | NKG7 |
| PARVA | DDX3X | SART2 | CD3Z |
| --- | H3F3A | DAB2 | IL2RG |
| PPP2R5D | H3F3A /// LOC440926 | CLIC2 | CD38 |

(continued)

| OAZ1 | PPIA | CLEC2B | CXCL13 |
|---|---|---|---|
| ZNF337 | RPS18 | PRRX1 | CD19 |
| TMEM4 | RPL41 | IFI16 | BANK1 |

Table 6 Examples of correlating genes

| DHRS2 | ERBB2 | H2AFZ | IGHG1 |
|---|---|---|---|
| CXorf40A /// CXorf40B | PERLD1 | MAD2L1 | APOL5 |
| DEGS1 | STARD3 | CDC2 | RARB |
| ALDH3B2 | GRB7 | CCNB1 | CLDN18 |
| SLC9A3R1 | CRK7 | CCNB2 | HBZ |
| INPP4B | PPARBP | CENPA | MUC3A |
| TP53AP1 | CASC3 | KPNA2 | --- |
| EMP2 | PSMD3 | ASPM | APOC4 |
| CACNG4 | PNMT | CDCA8 | ACRV1 |
| SULT2B1 | THRAP4 | KIF11 | FSHR |
| DEK | WIRE | CCNA2 | SPTA1 |
| DHCR24 | LOC339287 | ECT2 | EPC1 |
| RBM34 | PCGF2 | PTTG 1 | MYO15A |
| SLC38A1 | GSDML | BUB1 | GP1BB |
| AGPS | PIP5K2B | MELK | OR2B2 |
| CXorf40B | RPL19 | RRM2 | ENO1 |
| MSX2 | PPP1R10 | TPX2 | TCF21 |
| STC2 | LASP1 | DLG7 | GYPB |
| C14orf10 | SPDEF | MLF1IP | WNT6 |
| CREG1 | PSMB3 | STK6 | ASH1L |
| JMJD2B | GPC1 | BM039 | RPL37A |

Table 7 Examples of correlating genes

| IGKC | KCTD3 | MLPH | MMP1 |
|---|---|---|---|
| --- | TSNAX | FOXA1 | SLC16A3 |
| IGJ@ /// IGLC1 /// IGLC2 /// IGLV3-25 /// 1GLV2-14 | C1orf22 | SPDEF | KIAA1199 |
| IGLC2 | GATA3 | GATA3 | CTSB |
| IGKC /// IGKV1-5 | LGALS8 | AGR2 | SLAMF8 |
| LOC391427 | FOXA1 | CA12 | CORO1C |
| IGL@ /// IGLC1 /// IGLC2 /// IGLV3-25 /// IGLV2-14 /// IGLJ3 | MCP | ESR1 | PLAU |
| IGKV1D-13 | SSA2 | KIAA0882 | AQP9 |
| IGLV2-14 | I L6ST | SCNN1A | PDGFD |
| LOC339562 | GGPS1 | XBP1 | RGS5 |

(continued)

| IGKC | KCTD3 | MLPH | MMP1 |
|---|---|---|---|
| IGKV1-5 | CCNG2 | RHOB | PLAUR |
| IGLJ3 | DHX29 | FBP1 | CHST11 |
| LOC91353 | ZNF281 | GALNT7 | SOD2 |
| IGHA1 /// IGHD /// IGHG1 /// IGHM /// LOC390714 | FLJ20273 | MYO5C | TREM1 |
| LOC91316 | KIAA0882 | TFF3 | HN1 |
| IGHM | C1orf25 | CELSR1 | MRPS14 |
| IGHA1 /// IGHG1 /// IGHG3 /// LOC390714 | ABAT | LOC400451 | ACTR3 |
| IGH@ /// IGHG1 /// IGHG2 /// IGHG3 /// IGHM | HNRPH2 | SLC44A4 | RIPK2 |
| IGH@ /// IGHA1 /// IGHA2 /// IGHD /// IGHG1 /// IGHG2 /// IGHG3 /// IGHM /// MGC27165 /// LOC390714 | MRPS14 | MUC1 | ECHDC2 |
| IGJ | KIAA0040 | KIAA1324 | GBP1 |
| POU2AF1 | ERBB2IP | KRT18 | RRM2 |

Table 8 Examples of correlating genes

| PGR | SOX4 | TOP2A | UBE2C | ESR1 | VEGF |
|---|---|---|---|---|---|
| IL6ST | MARCKSL1 | TPX2 | BIRC5 | CA12 | ESM1 |
| MAPT | DSC2 | KIF11 | TPX2 | GATA3 | FLT1 |
| GREB1 | HOMER3 | CDC2 | STK6 | KIAA0882 | COL4A1 |
| ABAT | TMSB10 | ASPM | CCNB2 | MLPH | LSP1 |
| SCUBE2 | TCF3 | NUSAP1 | KIF2C | IL6ST | EPOR |
| NAT1 | ZNF124 | KIF4A | CDC20 | FOXA1 | COL4A2 |
| LRIG1 | PCAF | KIF20A | PTTG1 | SLC39A6 | PTGDS |
| SLC39A6 | PTMA | CCNB2 | PRC1 | C6orf97 | ENTPD1 |
| RBBP8 | IGSF3 | BIRC5 | NUSAP1 | C6orf211 | BNIP3 |
| SIAH2 | ENC1 | C10orf3 | C10orf3 | MYB | TPST1 |
| ARL3 | MTF2 | UBE2C | CENPA | ANXA9 | GLIPR1 |
| C9orf116 | E2F3 | SPAG5 | KIF4A | FBP1 | ZNFN1A1 |
| CA12 | TGIF2 | STK6 | RACGAP1 | SCNN1A | PCDH7 |
| MGC35048 | DBN1 | CCNB1 | ZWINT | MAPT | RGS13 |
| STC2 | DSP | NEK2 | PSF1 | NAT1 | GAS7 |
| MEIS4 | KLHL24 | RACGAP1 | BUB1 B | CELSR1 | LOC56901 |
| ADCY1 | PPP1R14B | KIF2C | DLG7 | PH-4 | TLR4 |
| C6orf97 | OPN3 | PTTG1 | FOXM1 | EVL | SYNCRIP |
| ESR1 | HSPA5BP1 | MKI67 | LOC146909 | XBP1 | EVI2A |
| NME5 | CREBL2 | MAD2L1 | ESPL1 | AGR2 | FNBP3 |

Table 9 Examples of correlating genes+

| EIF4B | NAT1 | CA12 | RACGAP1 | DCN |
|---|---|---|---|---|
| IMPDH2 | PSD3 | ESR1 | UBE2C | FBLN1 |
| NACA | EVL | GATA3 | NUSAP1 | GLT8D2 |
| RPL13A | ESR1 | SCNN1A | STK6 | SERPINF1 |
| RPL29 | KIAA0882 | MLPH | PSF1 | PDGFRL |
| RPL14 /// RPL14L | MAPT | FOXA1 | CCNB2 | CXCL12 |
| ATP5G2 | C9orf116 | IL6ST | ZWINT | CRISPLD2 |
| GLTSCR2 | ASAH1 | KIAA0882 | LOC146909 | CTSK |
| RPL3 | PCM1 | ANXA9 | BIRC5 | FSTL1 |
| TINP1 | SCUBE2 | BHLHB2 | PRC1 | SFRP4 |
| RPL15 | IL6ST | XBP1 | C10orf3 | FBN1 |
| QARS | ABAT | AGR2 | TPX2 | SPARC |
| LETMD1 | MLPH | MAPT | KIF11 | CDH11 |
| PFDN5 | VAV3 | JMJD2B | DLG7 | FAP |
| EEF2 | C14orf45 | RHOB | TOP2A | SPON1 |
| RPL6 | FOXA1 | CELSR1 | MELK | C1S |
| RPL29 /// LOC283412 /// LOC284064 /// LOC389655 /// LOC391738 /// LOC401911 | GATA3 | SPDEF | CENPA | PRRX1 |
| RPL18 | KIF13B | VGLL1 | NEK2 | RECK |
| EEF1B2 | CA12 | KRT18 | KIF2C | CSPG2 |
| RPL10A | MUC1 | C1orf34 | CCNB1 | LUM |
| RPS9 | C4A /// C4B | WWP1 | KIF20A | ANGPTL2 |

Table 10 Examples of correlating genes

| CTSB | IGFBP3 | KRT17 | GABRP | FBXO28 | KIAA0101 |
|---|---|---|---|---|---|
| IFI30 | VIM | KRT14 | SOX10 | PARP1 | NUSAP1 |
| FCER1 G | EFEMP2 | KRT5 | SFRP1 | EPRS | RRM2 |
| NPL | C1R | KRT6B | ROPN1B | IARS2 | CCNB2 |
| LAPTM5 | GAS1 | TRIM29 | KRT5 | CGI-115 | ZWINT |
| FCGR1A | PLS3 | MIA | MIA | C1orf37 | PRC1 |
| CD163 | SNAI2 | DST | MMP7 | TFB2M | DTL |
| TYROBP | SERPING1 | ACTG2 | KRT17 | WDR26 | TPX2 |
| NCF2 | CFH /// CFHL1 | SFRP1 | DMN | RBM34 | KIF11 |
| FCGR2A | ID3 | MYLK | KRT6B | FH | C10orf3 |
| ITGB2 | CFH | GABRP | BBOX1 | POGK | CDC2 |
| LILRB1 | ENPP2 | S100A2 | VGLL1 | NVL | NEK2 |
| OLR1 | FSTL1 | SOX10 | BCL11A | TIMM17A | ASF1 B |
| C1QB | NXN | ANXA8 | TRIM29 | ADSS | BIRC5 |
| ATP6V1B2 | C10orf10 | DMN | CRYAB | CACYBP | KIF4A |

(continued)

| CTSB | IGFBP3 | KRT17 | GABRP | FBXO28 | KIAA0101 |
|---|---|---|---|---|---|
| FCGR1A /// LOC440607 | FBLN1 | BBOX1 | SERPINB5 | CNIH4 | BUB1 B |
| SLC16A3 | NNMT | SERPINB5 | SOSTDC1 | GGPS1 | KIF20A |
| MSR1 | C1S | KCNMB1 | NFIB | DEGS1 | UBE2C |
| PLAUR | IF116 | DSG3 | ELF5 | FAM20B | MLF1IP |
| CHST11 | NRN1 | DSC3 | KRT14 | MRPS14 | TOP2A |
| FTL | PDGFRA | KLK5 | ANXA8 | TBCE | C22orf18 |

Table 11 Examples of correlating genes

| CHPT1 | PCNA | CCND1 | NEK2 | NR2F2 | PDLIM5 |
|---|---|---|---|---|---|
| SGK3 | PSF1 | CA12 | ASPM | SORBS1 | CRSP8 |
| STC2 | MAD2L1 | TLE3 | DTL | IGF1 | RSL1D1 |
| PKP2 | RAD51 AP1 | SLC39A6 | CENPF | AOC3 | FZD1 |
| CCNG2 | CDC2 | ESR1 | NUSAP1 | LHFP | PUM1 |
| SP110 | MLF1IP | PPFIA1 | TPX2 | ABCA8 | FAM63B |
| ACADM | H2AFZ | MAGED2 | CCNB2 | GNG11 | DCTD |
| GCHFR | TPX2 | FN5 | C10orf3 | ADH1B | APP |
| ABCD3 | CCNE2 | WWP1 | KIF20A | FHL1 | --- |
| IL6ST | RACGAP1 | C10orf116 | UBE2C | MEOX2 | DXS9879E |
| TSPAN6 | MCM2 | JMJD2B | TOP2A | C5orf4 | HFE |
| WDR26 | KIF11 | FBP1 | CDC2 | PPAP2A | GLRB |
| CELSR3 | CCNB1 | UBE2E3 | BIRC5 | COL14A1 | MRPS18A |
| TFCP2L1 | DLG7 | AGR2 | KIAA0101 | CAV1 | BMPR1 B |
| STXBP3 | CDCA8 | FOXA1 | FOXM1 | LPL | SAV1 |
| NAP1L1 | NUSAP1 | FADD | RRM2 | P2RY5 | TROAP |
| MYBPC1 | STK6 | TEGT | RACGAP1 | FABP4 | RPS2 /// LOC91561 /// LOC148430 /// LOC286444 /// LOC400963 /// LOC440589 |
| DSG2 | CCNB2 | COPZ1 | KIF11 | CHRDL1 | TOMM40 |
| OSBPL1A | RNASEH2A | MRPS30 | PRC1 | ELK3 | ITGAV |
| SEC14L2 | MELK | KRT18 | CCNB1 | C10orf56 | ESPL1 |
| ARL1 | ZWINT | FKBP4 | ZWINT | ITM2B | MAP4K5 |

Table 12 Examples of correlating genes

| PRC1 | FHL1 |
|---|---|
| NUSAP1 | CHRDL1 |
| CCNB2 | FABP4 |
| BIRC5 | AOC3 |
| UBE2C | ADH1B |

(continued)

| PRC1 | FHL1 |
|---|---|
| FLJ10719 | G0S2 |
| TPX2 | CAV1 |
| BUB1B | ITIH5 |
| FOXM1 | ADIPOQ |
| C10orf3 | LHFP |
| KIF11 | ABCA8 |
| KIF2C | GPX3 |
| KIF4A | PLIN |
| LOC146909 | DPT |
| ZWINT | TNS1 |
| CENPA | LPL |
| PTTG1 | GPD1 |
| DLG7 | SRPX |
| STK6 | RBP4 |
| KIAA0101 | CIDEC |
| RACGAP1 | TGFBR2 |

In summary, the present invention is predicated on a method of identification of a panel of genes informative for the outcome of disease which can be combined into an algorithm for a prognostic or predictive test.

**Claims**

1. Method for predicting an outcome of breast cancer in a patient, said method comprising:

    (a) determining in a biological sample from said patient an expression level of the genes CHPT1, CXCL13, ESR1, IGKC, MLPH, MMP1, PGR, RACGAP1, and TOP2A;
    (b) based on the expression level of the plurality of genes determined in step (a) determining a risk score for each gene; and
    (c) mathematically combining said risk scores to yield a combined score, wherein said combined score is indicative of a prognosis of said patient.

2. Method of claim 1, wherein a threshold is determined for said combined score and discriminated into high and low risk by applying the threshold on the combined score.

3. Method of any one of the preceding claims wherein said prognosis is the determination of the risk of recurrence of cancer in said patient within 5 years or the risk of developing distant metastasis.

4. Method of any one of the preceding claims, wherein said prognosis is a classification of said patient into one of three distinct classes, said classes corresponding to a "high risk" class, an "intermediate risk" class and a "low risk" class.

5. Method of any one of the preceding claims, wherein said determination of expression levels is in a formalin-fixed paraffin embedded sample or in a fresh-frozen sample.

6. Method of any one of the preceding claims, comprising the additional steps of:

    (d) classifying said sample into one of at least two clinical categories according to clinical data obtained from said patient and/or from said sample, wherein each category is assigned to at least one of said genes of step (a) ; and
(e) determining for each clinical category a risk score;
wherein said combined score is obtained by mathematically combining said risk scores of each patient.

**7.** Method of claim 6 wherein said clinical data comprises at least one gene expression level.

**8.** Method of claim 7, wherein said gene expression level is a gene expression level of at least one of the genes of step (a).

**9.** Method of any of claims 6 to 8, wherein step (d) comprises applying a decision tree.

FIG 1

FIG 2

**EP 2 469 440 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **GLAS A.M. et al.** Converting a breast cancer microarray signature into a high-throughput diagnostic test. *BMC Genomics,* 30 October 2006, vol. 7, 278

- **ESTEVA FT et al.** Prognostic role of a multigene reverse transcriptase-PCR assay in patients with node-negative breast cancer not receiving adjuvant systemic therapy. *Clin Cancer Res,* 2005, vol. 11, 3315-3319
- Molecular Cloning. A laboratory manual. Cold Spring Harbour Laboratory Press, 1989